(19) Europäisches Patentamt
European Patent Office
Office européen des brevets

(11) EP 1 757 603 A1

(12) EUROPEAN PATENT APPLICATION
published in accordance with Art. 158(3) EPC

(43) Date of publication:
**28.02.2007 Bulletin 2007/09**

(21) Application number: **05739114.6**

(22) Date of filing: **11.05.2005**

(51) Int Cl.:
***C07D 417/12*** (2006.01) ***A61K 9/06*** (2006.01)
***A61K 9/08*** (2006.01) ***A61K 31/4439*** (2006.01)
***A61P 27/02*** (2006.01) ***A61P 27/04*** (2006.01)
***A61P 27/14*** (2006.01)

(86) International application number:
**PCT/JP2005/008584**

(87) International publication number:
**WO 2005/108396 (17.11.2005 Gazette 2005/46)**

(84) Designated Contracting States:
**AT BE BG CH CY CZ DE DK EE ES FI FR GB GR HU IE IS IT LI LT LU MC NL PL PT RO SE SI SK TR**

(30) Priority: **11.05.2004 JP 2004141012**

(71) Applicant: **SANTEN PHARMACEUTICAL CO., LTD.**
**Higashiyodogawa-ku,**
**Osaka-shi,**
**Osaka 533-8651 (JP)**

(72) Inventors:
- **NAKAMURA, Masatsugu**
  **Ikoma-shi,**
  **Nara 6300101 (JP)**
- **HIRAI, Shin-ichiro**
  **Ikoma-shi,**
  **Nara 6300101 (JP)**

(74) Representative: **Müller-Boré & Partner**
**Patentanwälte**
**Grafinger Straße 2**
**D-81671 München (DE)**

# (54) THERAPEUTIC AGENT FOR KERATOCONJUNCTIVA DISORDER

(57) An object of the present invention is to search a new medicinal use of 5-[p-[2-(methyl-2-pyridylamino) ethoxy]benzyl]-2,4-thiazolidinedione and 5-[[4-[2-(5-ethyl-2-pyridinyl)ethoxy]phenyl]methyl]-2,4-thiazolidinedione. 5-[p-[2-(methyl-2-pyridylamino)ethoxy]benzyl]-2, 4-thiazolidinedione, 5-[[4-[2-(5-ethyl-2-pyridinyl)ethoxy] phenyl]methyl]-2,4-thiazolidinedione or a salt thereof exerts an excellent improving effect on corneal disorder models, therefore, it is useful as a therapeutic agent for a keratoconjunctival disorder such as dry eyes, corneal ulcer, keratitis or conjunctivitis.

EP 1 757 603 A1

**Description**

Technical Field

The present invention relates to a therapeutic agent for a keratoconjunctival disorder such as dry eyes, corneal ulcer, keratitis or conjunctivitis, comprising as an active ingredient, 5-[p-[2-(methyl-2-pyridylamino)ethoxy]benzyl]-2,4-thiazolidinedione, 5-[[4-[2-(5-ethyl-2-pyridinyl)ethoxy]phenyl]methyl]-2,4-thiazolidinedione, both of which are a thiazolidinedione derivative, or a salt thereof.

Background Art

Cornea is a transparent avascular tissue having a diameter of about 1 cm and a thickness of about 1 mm, while conjunctiva is a mucosal membrane covering the eyeball surface posterior to the corneal margin, and the back face of the eyelid. The cornea and the conjunctiva are known to significantly affect the visual function. Keratoconjunctival disorders caused due to a variety of diseases such as corneal ulcer, keratitis, conjunctivitis, dry eyes and the like may adversely affect normal architecture of epithelium, and furthermore, may impair structures and functions of the stroma and endothelium, when the repair of these disorders is retarded, alternatively when these disorders are prolonged without making repair on some grounds. That is because the cornea and the conjunctiva are connected tissues. In these years, with the development of cell biology, factors participating in cell proliferation, migration, adhesion, extension, differentiation and the like had been elucidated, and it was reported that these factors play important roles in repair of corneal disorders (Japanese Review of Clinical Ophthalmology, 46, 738-743 (1992) and Ophthalmic Surgery, 5, 719-727 (1992)).
On the other hand, Japanese Patent No. 2614497 describes the invention which relates to a substituted thiazolidinedione derivative such as 5-[p-[2-(methyl-2-pyridylamino)ethoxy]benzyl]-2,4-thiazolidinedione (generic name: rosiglitazone), and that such a compound is useful as a therapeutic agent or a preventive agent for hyperglycemia. Further, JP-A-61-267580 describes the invention which relates to a thiazolidinedione derivative such as 5-[[4-[2-(5-ethyl-2-pyridinyl)ethoxy]phenyl]methyl]-2,4-thiazolidinedione (generic name: pioglitazone), and that such a compound has an action of lowering glucose levels and lipid levels in the blood and is useful as a therapeutic agent for diabetes. International Publication WO 02/13812 discloses that an insulin-resistance improving agent such as rosiglitazone or pioglitazone is useful as a therapeutic agent for an inflammatory disease.
However, there has been no report in which a pharmacological action of these compounds on an eye disease such as a keratoconjunctival disorder is studied.

Disclosure of the invention

It is an interesting subject to search a new medicinal use of 5- [p- [2- (methyl-2-pyridylamino)ethoxy]benzyl]-2,4-thiazolidinedione and 5-[[4-[2-(5-ethyl-2-pyridinyl)ethoxy]phenyl]methyl]-2,4-thiazolidinedione, both of which are thiazolidinedione derivatives.
The present inventors have made intensive studies in order to search a new medicinal use of the above-mentioned compounds, and as a result, they found that the above-mentioned compounds and salts thereof exert an excellent improving effect on a corneal damage in a test for therapeutic effect using corneal disorder models, and thus the present invention has been accomplished.
That is, the present invention is directed to a therapeutic agent for a keratoconjunctival disorder such as dry eyes, corneal ulcer, keratitis or conjunctivitis, comprising as an active ingredient, 5-[p-[2-(methyl-2-pyridylamino)ethoxy] benzyl]-2,4-thiazolidinedione (hereinafter referred to as "rosiglitazone"), 5-[[4-[2-(5-ethyl-2-pyridinyl)ethoxy]phenyl]methyl]-2,4-thiazolidinedione (hereinafter referred to as "pioglitazone") or a salt thereof.
Rosiglitazone and pioglitazone of the present invention are thiazolidinedione derivatives represented by the following chemical structural formulae, respectively.

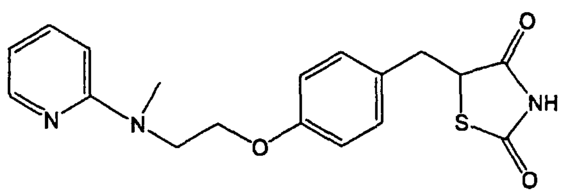

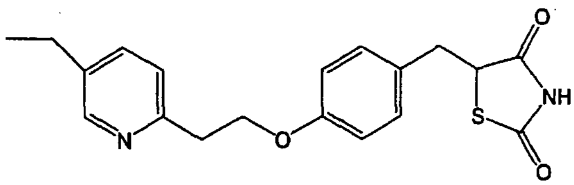

The salt of rosiglitazone or pioglitazone of the present invention is not particularly limited as long as it is a pharmaceutically acceptable salt. Examples thereof include salts with an inorganic acid such as hydrochloric acid, nitric acid or sulfuric acid, salts with an organic acid such as acetic acid, fumaric acid, maleic acid, succinic acid or tartaric acid, and the like. More preferred salts are a hydrochloride and a maleate. Quaternary ammonium salts of rosiglitazone and pioglitazone of the present invention are also included in the salt according to the present invention. Incidentally, rosiglitazone or pioglitazone of the present invention may be in a form of a hydrate or a solvate. Further, a geometric isomer, optical isomer, tautomer or polymorphism of rosiglitazone or pioglitazone may also be included in the scope of the present invention.

The keratoconjunctival disorder referred to herein means the state of damaged cornea and/or conjunctiva due to various factors, and examples thereof include dry eyes, corneal ulcer, keratitis, conjunctivitis and the like.

The therapeutic agent for a keratoconjunctival disorder of the invention may be administered either orally or parenterally. Examples of the dosage form include eye drops, ophthalmic ointments, injections, tablets, capsules, granules, powders and the like. In particular, eye drops are preferred. These can be prepared using any of generally used techniques. For example, the eye drops can be prepared using a tonicity agent such as sodium chloride or concentrated glycerin, a buffer such as sodium phosphate or sodium acetate, a surfactant such as polyoxyethylene sorbitan monooleate, polyoxyl 40 stearate or polyoxyethylene hydrogenated castor oil, a stabilizer such as sodium citrate or sodium edetate, a preservative such as benzalkonium chloride or paraben as needed. The pH of the eye drops is permitted as long as it falls within the range that is acceptable as an ophthalmic preparation, but is preferably in the range of from 4 to 8.

The ophthalmic ointment can be prepared with a generally used base such as white soft paraffin or liquid paraffin. Also, oral preparations such as tablets, capsules, granules and powders can be prepared by adding an extender such as lactose, crystalline cellulose, starch or vegetable oil, a lubricant such as magnesium stearate or talc, a binder such as hydroxypropyl cellulose or polyvinyl pyrrolidone, a disintegrant such as carboxymethyl cellulose calcium or low-substituted hydroxypropylmethyl cellulose, a coating agent such as hydroxypropylmethyl cellulose, macrogol or a silicone resin, a film forming agent such as gelatin film, and the like, as needed.

The dose can properly be selected depending on the symptoms, age, dosage form and the like. In the case of an eye drop, it may be instilled once to several times a day at a concentration of from 0.0001 to 1% (w/v), preferably from 0.001 to 1% (w/v). In the case of an oral preparation, it may be administered once or divided into several times at a dose of generally from 0.1 to 5000 mg per day, preferably from 1 to 1000 mg per day. Advantage of the Invention

As will be described below, when a test for a therapeutic effect on a corneal damage was carried out, both rosiglitazone and pioglitazone hydrochloride of the present invention were found to exert an excellent improving effect on corneal disorder models. Therefore they are useful as a therapeutic agent for a keratoconjunctival disorder such as dry eyes, corneal ulcer, keratitis or conjunctivitis.

Best Mode for Carrying Out the Invention

Hereinafter, results of a pharmacological test and preparation examples will be shown, however, these examples are for understanding the invention well, and are not meant to limit the scope of the invention.

[Pharmacological test]

Test for therapeutic effect on corneal damage

Using male SD rats, in accordance with the method of Fujihara et al. (Invest. Ophthalmol. Vis. Sci. 42 (1) : 96-100 (2001)), corneal disorder models were produced. After the production of the corneal disorder models, the improvement ratio of a corneal damage was evaluated by a method (Journal of the eye 21 (1) : 87-90 (2004)) modified from the method of Miyata et al. (Japanese Review of Clinical Ophthalmology 48 (2) : 183-188 (1994)).

(Test method)

A male SD rat was systemically anesthetized by an administration of Nembutal. Subsequently the exorbital lacrimal gland was removed and a corneal damage was induced over a period of 2 months.
Then, the present compound (rosiglitazone or pioglitazone hydrochloride) was administered as follows.

Rosiglitazone instillation group:

A physiological saline solution of rosiglitazone (0.02%) was instilled into both eyes 6 times a day for 14 days (one group consisting of 8 animals).

Pioglitazone instillation group:

A physiological saline solution of pioglitazone hydrochloride (0.01%) was instilled into both eyes 6 times a day for 14 days (one group consisting of 8 animals).
In a control group, physiological saline was instilled into both eyes 6 times a day for 14 days (one group consisting of 8 animals).
Fourteen days after the start of instillation, the damaged parts of the cornea were stained with fluorescein. For each of the upper, middle and lower parts of the cornea, the degree of fluorescein staining was evaluated by scoring according to the criteria shown below and the improvement ratio of corneal damage was calculated from the mean value of the total scores for each of the above-mentioned parts.

(Evaluation criteria)

0: No punctate staining
1: Scattered staining (punctate staining being separated)
2: Moderate staining (a part of punctate staining being adjacent)
3: Heavy staining (punctate staining being barely separated)

(Results)

By taking the mean value of the total scores for the control group (physiological saline) as a standard (improvement ratio: 0%) and according to the calculation formula shown below, the improvement ratios for rosiglitazone (0.02%) instillation group and pioglitazone (0.01%) instillation group were calculated, which are shown in Table 1. The mean value of the scores is a mean of those of 8 cases, respectively.

Improvement ratio (%) = {(control) − (the present compound)} / damage degree × 100

Damage degree = (control) − (normal eye)

[Table 1]

| Group | Mean value of total scores | Improvement ratio (%) |
|---|---|---|
| Normal eye | 2.9 | - |
| Control group | 6.1 | - |
| Rosiglitazone (0.02%) instillation group | 3.9 | 68.7 |

(continued)

| Group | Mean value of total scores | Improvement ratio (%) |
|---|---|---|
| Pioglitazone (0.01%) instillation group | 4.1 | 62.5 |

(Discussion)

As apparent from the results of the above pharmacological test using rats, rosiglitazone and pioglitazone significantly improve a corneal damage.

[Preparation examples]

Hereinafter, representative formulation examples using rosiglitazone or pioglitazone will be shown.

| | |
|---|---|
| Preparation example 1 | |
| In 100 ml, | |
| Rosiglitazone | 10 mg |
| Sodium Chloride | 900 mg |
| Sterile purified water | q.s. |

By altering the amount of rosiglitazone to be added, an eye drop at a concentration of 0.001% (w/v), 0.03% (w/v), 0.1% (w/v), 0.30 (w/v), 1.0% (w/v), or 3.0% (w/v) can be prepared.

| | |
|---|---|
| Preparation example 2 | |
| In 100 ml, | |
| Pioglitazone hydrochloride | 100 mg |
| Sodium Chloride | 800 mg |
| Disodium hydrogen phosphate | 100 mg |
| Sodium dihydrogen phosphate | q.s. |
| Sterile purified water | q.s. |

By altering the amount of pioglitazone hydrochloride to be added, an eye drop at a concentration of 0.05% (w/v), 0.3% (w/v), 0.5% (w/v), 1.5% (w/v), or 3% (w/v) can be prepared.

| | |
|---|---|
| Preparation example 3 | |
| In 100 g, | |
| Rosiglitazone | 0.3 g |
| Liquid paraffin | 10.0 g |
| White soft paraffin | q.s. |

By altering the amount of rosiglitazone to be added, an ophthalmic ointment at a concentration of 1% (w/w) or 3% (w/w) can be prepared.

## Claims

1. A use of 5-[p-[2-(methyl-2-pyridylamino)ethoxy]benzyl]-2,4-thiazolidinedione, 5-[[4-[2-(5-ethyl-2-pyridinyl)ethoxy] phenyl]methyl]-2,4-thiazolidinedione or a salt thereof for manufacturing a therapeutic agent for a keratoconjunctival disorder.

2. The use according to claim 1, wherein the keratoconjunctival disorder is dry eyes, corneal ulcer, keratitis or conjunctivitis.

3. The use according to claim 1, wherein the therapeutic agent is in a dosage form of an eye drop or an ophthalmic ointment.

**INTERNATIONAL SEARCH REPORT**

International application No.
PCT/JP2005/008584

A. CLASSIFICATION OF SUBJECT MATTER
Int.Cl[7] C07D417/12, A61K9/06, 9/08, 31/4439, A61P27/02, 27/04, 27/14,

According to International Patent Classification (IPC) or to both national classification and IPC

B. FIELDS SEARCHED

Minimum documentation searched (classification system followed by classification symbols)
Int.Cl[7] C07D404/00-421/14, A61K9/00-9/08, 31/00-31/80, A61P27/00-27/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
MEDLINE(STN), EMBASE(STN), BIOSIS(STN), BIOTECHABS(STN), CAplus(STN), REGISTRY(STN), WPI(DIALOG), JSTPLUS(JOIS), JMEDPLUS(JOIS)

C. DOCUMENTS CONSIDERED TO BE RELEVANT

| Category* | Citation of document, with indication, where appropriate, of the relevant passages | Relevant to claim No. |
|---|---|---|
| X | WO 02/13812 A1 (PERSHADSINGH, Harrihar, A.), 21 February, 2002 (21.02.02), Full text & AU 200188271 A | 1-3 |
| Y | JP 2004-509961 A (Dr. Reddy's Research Foundation), 02 April, 2004 (02.04.04), Full text & AU 200191232 A & EP 1322647 A1 & NO 200301356 A & BR 200114196 A & HU 200301161 A2 & KR 2003082541 A & US 2004/0068116 A1 & CZ 200300864 A3 & ZA 200303223 A & MX 2003002580 A1 & NZ 525498 A & CN 1535271 A & SK 200300375 A3 | 1-3 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 August, 2005 (04.08.05) | 23 August, 2005 (23.08.05) |
| Name and mailing address of the ISA/ Japanese Patent Office | Authorized officer |
| Facsimile No. | Telephone No. |

Form PCT/ISA/210 (second sheet) (January 2004)

INTERNATIONAL SEARCH REPORT

International application No.
PCT/JP2005/008584

C (Continuation). DOCUMENTS CONSIDERED TO BE RELEVANT

| Category* | Citation of document, with indication, where appropriate, of the relevant passages | Relevant to claim No. |
|---|---|---|
| Y | JP 11-279062 A (Fujisawa Pharmaceutical Co., Ltd.), 12 October, 1999 (12.10.99), Full text & WO 99/38497 A2 & ZA 9900733 A & AU 9921844 A & EP 1004307 A2 & BR 9907715 A & NO 200003786 A & CZ 200002770 A3 & CN 1295476 A & KR 2001040457 A & EP 1161942 A2 & HU 200100214 A2 & US 6403598 B1 & MX 2000006882 A1 & US 2002/0161015 A1 & US 6476039 B1 | 1-3 |
| Y | Satoshi HOSOYA, "Tonyobyosei Kakumakusho", The Journal of the Eye, 1996, 13(6), pages 845 to 851, full text, particularly, "II Tonyobyosei Kakumakusho no Chiryo" | 1-3 |
| Y | Yasuichiro CHIKAMA, "Senensei Kakumaku Johi Kesson", Ganka, 2001, 43(12), pages 1625 to 1631, full text, particularly, "V. Senensei Kakumaku Johi Kesson no Chiryoho" | 1-3 |
| Y | Masataka HARINO et al., "Tonyobyosei Kakumakusho no 1 Rei to sono Kakumaku Naihi Saibo Shoken", Folia Ophthalmogica Japonica, 1989, 40, pages 2124 to 2130, full text | 1-3 |
| Y | Yasuko TAKANO et al., "Tonyobyo Kakumaku Johisho no 3 Rei", Japanese review of Clinical ophthalmology, 1998, 92(8), pages 1102 to 1105, full text | 1-3 |
| Y | Misao KISANUKI et al., "Jutoku na Kosaien o Tomonatta Tonyobyo Kakumakusho no 2 Rei", Folia Ophthalmogica Japonica, 1995, 46, pages 268 to 271, full text | 1-3 |
| Y | WATANABE, H. et al., Corneal Disorders in KKAy Mouse: A Type 2 Diabetes Model., Jpn. J. Ophthamol., 2002, 46, pages 130 to 139, full text | 1-3 |
| P,Y | Makiko HARA et al., "Idensei II gata Tonyobyo GK Rat ni okeru Kakumaku Johi Saibo no Zoshoku oyobi Bunka no Hen'i", Journal of Japanese Ophthalmological Society, 2005, 109(Special extra issue), page 272 | 1-3 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2614497 B
- JP 61267580 A
- WO 0213812 A


### Non-patent literature cited in the description


- *Japanese Review of Clinical Ophthalmology,* 1992, vol. 46, 738-743
- *Ophthalmic Surgery,* 1992, vol. 5, 719-727
- **FUJIHARA et al.** *Invest. Ophthalmol. Vis. Sci.,* 2001, vol. 42 (1), 96-100
- *Journal of the eye,* 2004, vol. 21 (1), 87-90
- **MIYATA et al.** *Japanese Review of Clinical Ophthalmology,* 1994, vol. 48 (2), 183-188